# EUROPEAN PATENT APPLICATION

(11) **EP 4 018 977 A2**
(43) Date of publication of application: **29.06.2022**
(21) Application number: 21215889.3
(22) Date of filing: 20.12.2021
(51) Int. Cl.: A61F 5/01, A43B 7/00, A43B 23/00

(54) **SHOE**

(30) Priority: 24.12.2020 JP 2020214743
(71) Applicant: ASICS Corporation, Kobe-shi Hyogo 650-8555 (JP)
(72) Inventor: MATSUNAGA, Ryo, Kobe-shi, Hyogo, 650-8555 (JP); ISHIKAWA, Tatsuya, Kobe-shi, Hyogo, 650-8555 (JP); YAMANE, Osamu, Kobe-shi, Hyogo, 650-8555 (JP); NAKAYA, Seigo, Kobe-shi, Hyogo, 650-8555 (JP)
(74) Representative: Becker, Eberhard

(57) **Abstract**

A shoe (1) includes: an upper (2) in which an upper middle foot portion (26) covering a middle foot portion of a foot of a wearer, an upper rear foot portion (27) covering a heel portion of the foot, and a foot insertion opening (20a) for inserting the foot are formed; a sole (3) located below the upper (2); a first support member (5) disposed below a medial longitudinal arch of the foot inside the upper (2) and including a lateral foot side portion fixed to the upper (2) or the sole (3); and a first pulling-up member (6) connected to a medial foot side portion of the first support member (5) and enabled to apply, to the first support member (5), force for pulling up the first support member (5) in a direction toward the upper rear foot portion (27) and away from the sole (3).

## Description

### BACKGROUND OF THE INVENTION

### 1. Field of the Invention

The present invention relates to a shoe including an upper and a sole.

### 2. Description of the Related Art

Medial tibial stress syndrome, so-called shin splint, is conventionally known that causes inflammation in a periosteum attached to a tibia by repeatedly performing motions such as running and jumping. The shin splint is classified into two types, an "anterior shin splint" in which inflammation occurs in a periosteum between a tibialis anterior tendon and a tibia due to excessive contraction of the tibialis anterior tendon, and a "posterior shin splint" in which inflammation occurs in a periosteum between a tibialis posterior tendon and a tibia due to excessive extension of the tibialis posterior tendon. It has been found that one of causes of occurrence of the posterior shin splint is falling of a medial longitudinal arch when a wearer's weight is applied to the wearer's foot.

Many techniques have conventionally been developed in which a reinforcing member is disposed inside an upper to suppress falling of the medial longitudinal arch by the reinforcing member, as in JP 2016-36703 A. However, development has not been progressed regarding a technique for reducing a burden on the tibialis posterior tendon for the purpose of prevention of the posterior shin splint.

Thus, it is desired to develop a shoe that can reduce the burden on the tibialis posterior tendon.

The present invention has been made in view of the above, and an object of the present invention is to obtain a shoe that can reduce the burden on the tibialis posterior tendon while suppressing the falling of the medial longitudinal arch.

### SUMMARY OF THE INVENTION

In order to solve the above-described problem and achieve the object, a shoe according to the present invention includes an upper in which an upper middle foot portion covering a middle foot portion of a foot of a wearer, an upper rear foot portion covering a heel portion of the foot, and a foot insertion opening for inserting the foot are formed, a sole located below the upper, a first support member disposed below a medial longitudinal arch of the foot inside the upper and including a lateral foot side portion fixed to the upper or the sole, and a first pulling-up member connected to a medial foot side portion of the first support member and enabled to apply, to the first support member, force for pulling up the first support member in a direction toward the upper rear foot portion and away from the sole.

### BRIEF DESCRIPTION OF THE DRAWINGS

FIG. 1 is a plan view schematically illustrating a state in which a skeleton model of a foot is overlapped on a shoe;
FIG. 2 is a perspective view of the shoe according to a first embodiment of the present invention;
FIG. 3 is a plan view of the shoe according to the first embodiment;
FIG. 4 is a side view of a medial foot side of the shoe according to the first embodiment;
FIG. 5 is a plan view schematically illustrating an outer shape of the shoe and a first support member and a second support member;
FIG. 6 is a cross-sectional view taken along line VI-VI illustrated in FIG. 4, and is a cross-sectional view illustrating a state before the first support member is pulled up;
FIG. 7 is a cross-sectional view illustrating a state after the first support member is pulled up from the state illustrated in FIG. 6;
FIG. 8 is a perspective view schematically illustrating a state in which the first support member is overlapped on a foot;
FIG. 9 is a cross-sectional view taken along line IX-IX illustrated in FIG. 4, and is a cross-sectional view illustrating a state before the second support member is pulled up;
FIG. 10 is a cross-sectional view illustrating a state after the second support member is pulled up from the state illustrated in FIG. 9;
FIG. 11 is a perspective view schematically illustrating a state in which the second support member is overlapped on the foot;
FIG. 12 is a perspective view of a shoe according to a second embodiment of the present invention;
FIG. 13 is a perspective view of a shoe according to a third embodiment of the present invention;
FIG. 14 is a plan view of the shoe according to the third embodiment;
FIG. 15 is a side view of a medial foot side of the shoe according to the third embodiment; and
FIG. 16 is a perspective view of a shoe according to a fourth embodiment of the present invention.

### DETAILED DESCRIPTION OF THE PREFERRED EMBODIMENTS

Hereinafter, examples of a shoe according to the present invention will be described in detail with reference to the drawings. Note that the present invention is not limited by the examples.

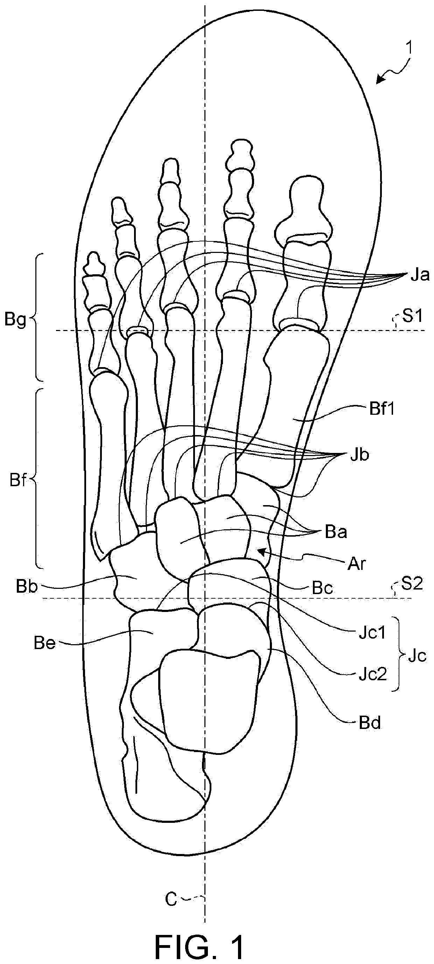

FIG. 1 is a plan view schematically illustrating a state in which a skeleton model of a foot is overlapped on a shoe 1. In embodiments described below, a direction in which a shoe center axis C, which is a perpendicular line passing through the center of the shoe 1 in a plan view of the shoe 1, extends is referred to as a front-rear direction, and a direction orthogonal to the front-rear direction in a plan view of the shoe 1 is referred to as a left-right direction.

In addition, in the front-rear direction, a direction from an end on a side where a portion covering a rear foot portion of the foot of the shoe 1 is located toward an end on a side where a portion covering a front foot portion of the foot of the shoe 1 is located is referred to as a front, and in the front-rear direction, a direction from the end on the side where the portion covering the front foot portion of the foot of the shoe 1 is located toward the end on the side where the portion covering the rear foot portion of the foot of the shoe 1 is located is referred to as a rear.

In addition, a median side in an anatomical position of the foot is referred to as a medial foot side, and a side opposite to the median side in the anatomical position of the foot is referred to as a lateral foot side. That is, a side closer to a median in the anatomical position is referred to as the medial foot side, and a side farther from the median in the anatomical position is referred to as the lateral foot side.

In addition, a height direction means a direction orthogonal to both the front-rear direction and the left-right direction unless otherwise specified, and a thickness means a dimension in the height direction unless otherwise specified.

The foot of a human body mainly includes a cuneiform bone Ba, a cuboid bone Bb, a scaphoid bone Bc, a talus bone Bd, a calcaneus bone Be, a metatarsal bone Bf, and a phalanx bone Bg. Joints of the foot include an MP joint Ja, a Lisfranc joint Jb, and a Chopart joint Jc. The Chopart joint Jc includes a calcaneocuboid joint Jc1 formed by the cuboid bone Bb and the calcaneus bone Be, and a talonavicular joint Jc2 formed by the scaphoid bone Bc and the talus bone Bd.

A middle foot portion of the foot refers to a portion from the MP joint Ja to the Chopart joint Jc. A medial longitudinal arch Ar of the foot refers to a portion from the calcaneus bone Be to a ball of the foot of a first metatarsal bone Bf1 via the talus bone Bd, the scaphoid bone Bc, and the medial cuneiform bone Ba, in the medial foot side of the foot.

In addition, in a case where a line along the left-right direction passing through a position corresponding to 25% to 50% of a dimension in the front-rear direction of the shoe 1 from a front end of the shoe 1 is defined as a first boundary line S1, and a line along the left-right direction passing through a position corresponding to 55% to 80% of a dimension in the front-rear direction of the shoe 1 from the front end of the shoe 1 is defined as a second boundary line S2, a portion located in front of the first boundary line S1 is a front foot portion of the shoe 1, a portion sandwiched between the first boundary line S1 and the second boundary line S2 is a middle foot portion of the shoe 1, and a portion located in the rear from the second boundary line S2 is a rear foot portion of the shoe 1. The first boundary line S1 is a line substantially along the MP joint Ja of a wearer with a standard body shape, and the second boundary line S2 is a line substantially along the Chopart joint Jc of the wearer with the standard body shape.

### (First embodiment)

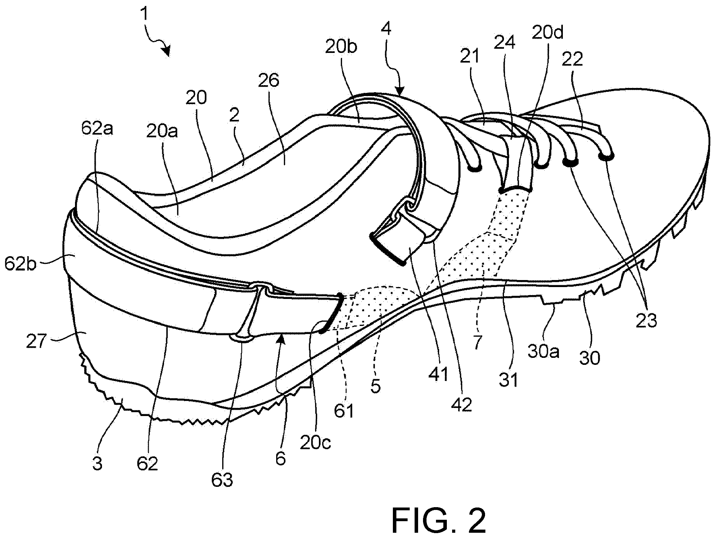

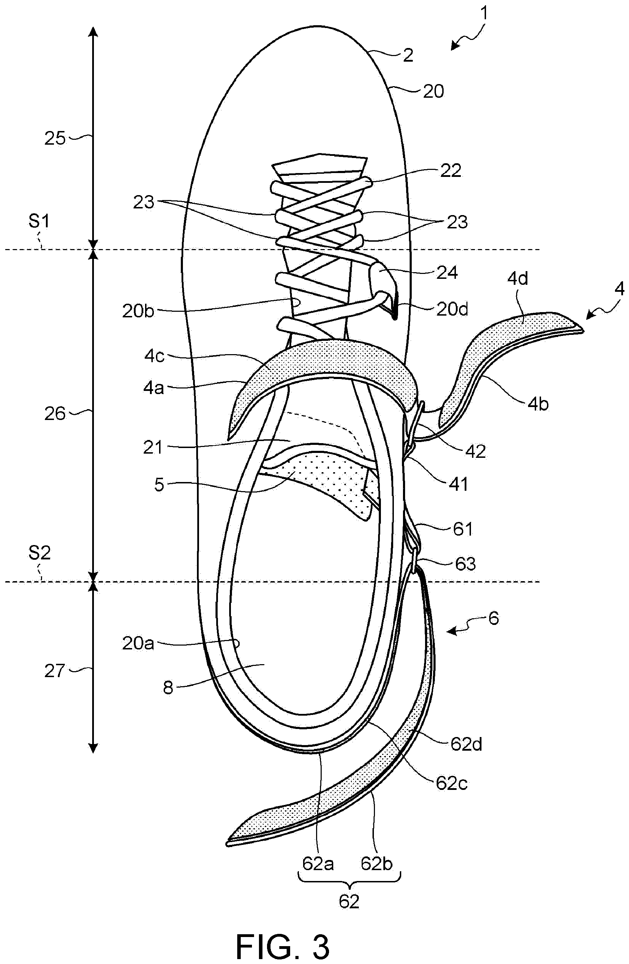

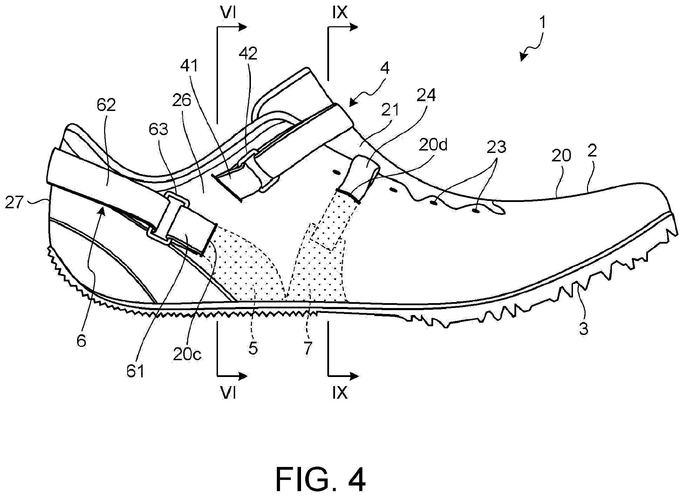

FIG. 2 is a perspective view of the shoe 1 according to a first embodiment of the present invention. FIG. 3 is a plan view of the shoe 1 according to the first embodiment. FIG. 4 is a side view of the medial foot side of the shoe 1 according to the first embodiment. FIGS. 2 to 4 illustrate only the shoe 1 for the left foot. Since the shoe 1 has a bilaterally symmetrical structure for the left foot and the right foot, only the shoe 1 for the left foot will be described in the present embodiment, and the description of the shoe 1 for the right foot will be omitted.

The shoe 1 is a spiked shoe for track and field in the present embodiment, but may be a running shoe, a shoe for sports such as soccer, basketball, volleyball, and the like. The shoe 1 includes an upper 2, a sole 3, an adjuster belt 4, a first support member 5, a first pulling-up member 6, and a second support member 7.

The upper 2 includes an upper main body 20, a shoe tongue 21, and a shoelace 22.

The upper main body 20 covers a portion on an instep side of the foot. A foot insertion opening 20a for inserting a foot of a wearer, and an opening 20b communicating with the foot insertion opening 20a and extending in front from the foot insertion opening 20a are formed at an upper portion of the upper main body 20. First string passing portions 23 and a second string passing portion 24 that are a plurality of string passing portions separated from each other in the front-rear direction are provided at left and right side edges of the opening 20b. The first string passing portions 23 are through holes penetrating the upper main body 20 in a vertical direction. One second string passing portion 24 of the plurality of string passing portions is provided at a medial foot side portion of the second support member 7. In the present embodiment, the second string passing portion 24 is formed by annularly folding back and stitching the medial foot side portion of the second support member 7, but may be formed by fixing an annular member separate from the second support member 7 to the medial foot side portion of the second support member 7.

As illustrated in FIG. 3, the upper main body 20 includes an upper front foot portion 25 that is a portion covering a front foot portion of the foot of the wearer with a standard body shape, an upper middle foot portion 26 that is a portion covering a middle foot portion of the foot of the wearer with the standard body shape, and an upper rear foot portion 27 that is a portion covering a heel portion of the foot of the wearer with the standard body shape. The upper front foot portion 25, the upper middle foot portion 26, and the upper rear foot portion 27 are connected together in this order in the front-rear direction from the front of the upper main body 20. The upper front foot portion 25 is located in front of the first boundary line S1. The upper middle foot portion 26 is located at a portion sandwiched between the first boundary line S1 and the second boundary line S2. The upper rear foot portion 27 is located in the rear from the second boundary line S2.

As illustrated in FIG. 2, a slit-shaped first through hole 20c and a slit-shaped second through hole 20d that communicate the inside and the outside of the upper 2 are formed in a medial foot side portion of the upper middle foot portion 26. The first through hole 20c is provided at a position overlapping the foot insertion opening 20a in the left-right direction. The second through hole 20d is provided at a position overlapping the opening 20b in the left-right direction.

As illustrated in FIG. 3, the shoe tongue 21 is a member for protecting the instep of the foot of the wearer. The shoe tongue 21 covers the opening 20b inside the upper main body 20. The shoe tongue 21 is fixed to the upper main body 20 by stitching, welding, bonding, or a combination thereof. As a material of the upper main body 20 and the shoe tongue 21, for example, a woven fabric, a knitted fabric, synthetic leather, or resin is used. In particular, in the shoe 1 in which air permeability and a lightweight property are required, it is preferable to use a double raschel warp knitted fabric knitted with polyester yarn as the material of the upper main body 20 and the shoe tongue 21. Note that the materials of the upper main body 20 and the shoe tongue 21 are not limited to the exemplified materials.

The shoelace 22 is a string-like member that alternately passes through the first string passing portions 23 and the second string passing portion 24 provided at one of the side edges of the opening 20b and the first string passing portions 23 provided at another of the side edges of the opening 20b, and is detachably attached to the upper main body 20.

As illustrated in FIG. 2, the sole 3 is located below the upper 2. The sole 3 covers a sole of the foot. The sole 3 includes an outsole 30 and a midsole 31. The sole 3 is fixed to the upper 2 by stitching, welding, bonding, or a combination thereof. A lower surface of the outsole 30 serves as a ground contact surface 30a that comes in contact with the ground. The midsole 31 is located on an upper surface of the outsole 30 and has a cushioning property. Note that the outsole 30 may be integrated with the midsole 31. The outsole 30 integrated with the midsole 31 is also referred to as a "unisole". In addition, as illustrated in FIG. 3, the sole 3 includes a footbed 8. The footbed 8 is placed on the outsole 30 with the midsole 31 interposed therebetween, inside the upper 2.

The sole 3 includes an insole (not illustrated) that covers a lower opening of the upper main body 20. The insole is fixed to an upper surface of the midsole 31 by bonding or welding. In addition, the insole is fixed to a lower edge of the upper main body 20 described above by stitching. In a case where the sole 3 includes the insole, the footbed 8 is placed on the outsole 30 with the insole and the midsole 31 interposed therebetween. Note that the sole 3 may have a structure in which the insole is omitted.

As illustrated in FIG. 2, the adjuster belt 4 is a member enabled to apply, to the upper main body 20, tightening force for pulling the medial foot side portion and a lateral foot side portion of the upper middle foot portion 26 toward each other. The adjuster belt 4 extends in the left-right direction across the opening 20b. The adjuster belt 4 is disposed near a boundary with the foot insertion opening 20a in the opening 20b in the front-rear direction. A fixed belt 41 and a connecting part 42 that support the adjuster belt 4 are provided in the medial foot side portion of the upper middle foot portion 26.

In the medial foot side portion of the upper middle foot portion 26, the fixed belt 41 is provided at a location that is in front of the first through hole 20c and is in the rear of the second through hole 20d. A first end of the fixed belt 41 is fixed to the upper middle foot portion 26. A second end of the fixed belt 41 is connected to the connecting part 42.

As illustrated in FIG. 3, a lateral foot side portion of the adjuster belt 4 is fixed to the lateral foot side portion of the upper middle foot portion 26, the adjuster belt 4 extends from the lateral foot side portion to the medial foot side portion of the upper middle foot portion 26, and then is folded back at the connecting part 42 and extends to the lateral foot side. The adjuster belt 4 includes a first tightening side extending portion 4a and a second tightening side extending portion 4b. A lateral foot side portion of the first tightening side extending portion 4a is fixed to the lateral foot side portion of the upper middle foot portion 26. The first tightening side extending portion 4a extends from the lateral foot side portion toward the medial foot side portion of the upper middle foot portion 26 to the connecting part 42. A hook-and-loop fastener 4c is attached to a surface of the first tightening side extending portion 4a facing away from the upper middle foot portion 26. A hook-and-loop fastener 4d is attached to a surface of the second tightening side extending portion 4b facing the upper middle foot portion 26. The second tightening side extending portion 4b can be fixed to and separated from the first tightening side extending portion 4a via the hook-and-loop fasteners 4c and 4d. In a state in which the second tightening side extending portion 4b is fixed to the first tightening side extending portion 4a, the second tightening side extending portion 4b extends from the connecting part 42 to the lateral foot side portion of the upper middle foot portion 26.

As illustrated in FIG. 2, the connecting part 42 connects the second end of the fixed belt 41 and a folded portion of the adjuster belt 4 to each other. A configuration of the connecting part 42 is not particularly limited as long as the fixed belt 41 and the adjuster belt 4 can be connected to each other, but is a ring-shaped member in the present embodiment. In the present embodiment, the second end of the fixed belt 41 passes through the connecting part 42 and is folded back, and overlapping portions of the fixed belt 41 are stitched together, whereby the fixed belt 41 is connected to the connecting part 42.

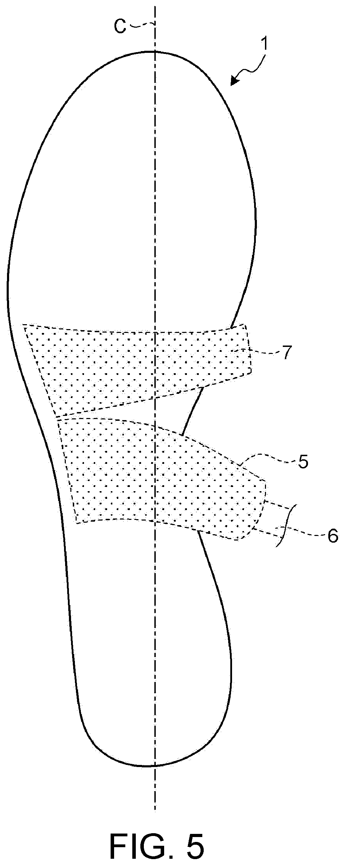

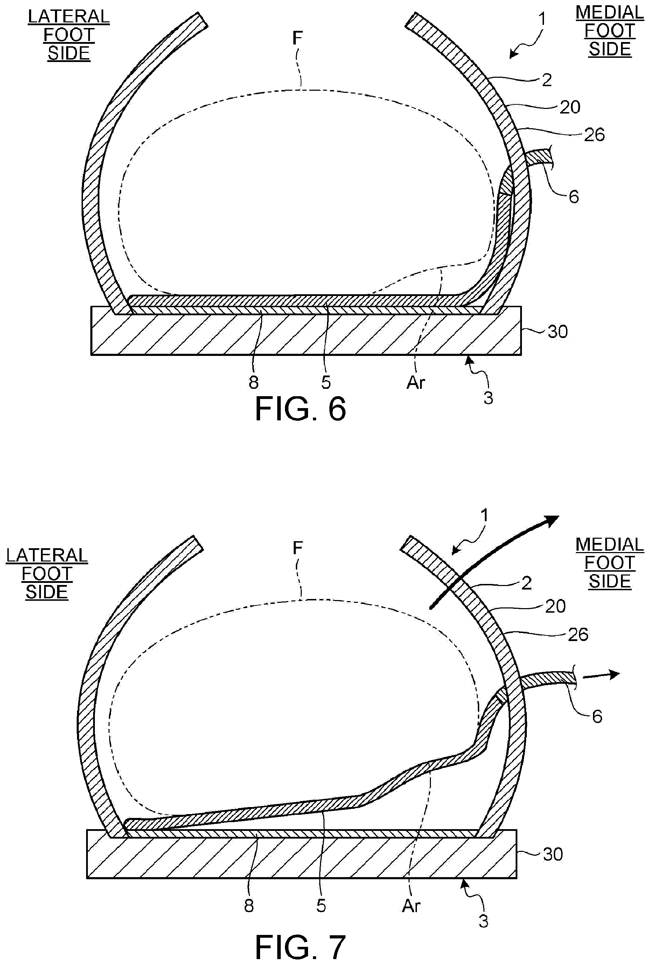

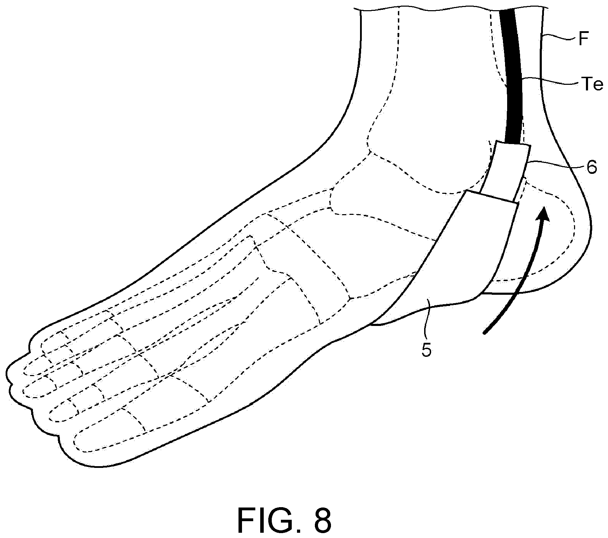

FIG. 5 is a plan view schematically illustrating an outer shape of the shoe 1 and the first support member 5 and the second support member 7. FIG. 6 is a cross-sectional view taken along line VI-VI illustrated in FIG. 4, and is a cross-sectional view illustrating a state before the first support member 5 is pulled up. FIG. 7 is a cross-sectional view illustrating a state after the first support member 5 is pulled up from the state illustrated in FIG. 6. FIG. 8 is a perspective view schematically illustrating a state in which the first support member 5 is overlapped on a foot F. As illustrated in FIG. 6, the first support member 5 is a member disposed below the medial longitudinal arch Ar of the foot F inside the upper 2, and its lateral foot side portion is fixed to the upper main body 20. The first support member 5 is disposed below the entire foot F including the medial longitudinal arch Ar. The first support member 5 is disposed in the upper middle foot portion 26. Note that the first support member 5 may be fixed to the footbed 8 or the insole of the sole 3. The first support member 5 is fixed to any of the upper main body 20, the footbed 8, and the insole by stitching, welding, bonding, or a combination thereof. As a material of the first support member 5, for example, a woven fabric, a knitted fabric, synthetic leather, or resin is used. As illustrated in FIG. 5, the width of the first support member 5 in the front-rear direction increases from a connection portion with the first pulling-up member 6 toward the lateral foot side. A plan view shape of the first support member 5 is not particularly limited, but is a trapezoidal shape in the present embodiment. A front end portion, a rear end portion, and a medial foot side end portion of the first support member 5 have a curved shape in the present embodiment, but may have, for example, a linear shape, a waveform shape, or a jagged shape.

As illustrated in FIG. 7, the first pulling-up member 6 is a member connected to a medial foot side portion of the first support member 5 and enabled to apply, to the first support member 5, force for pulling up the first support member 5 in a direction toward the upper rear foot portion 27 and away from the sole 3. In other words, as illustrated in FIG. 8, the first pulling-up member 6 extends along an extending direction of a tibialis posterior tendon Te of the foot F of the wearer, and is enabled to apply, to the first support member 5, force for pulling up the first support member 5 along the tibialis posterior tendon Te. In yet other words, the first pulling-up member 6 is enabled to apply, to the first support member 5, force for pulling up the first support member 5 obliquely upward and in the rear. As illustrated in FIG. 2, the first pulling-up member 6 passes through the medial foot side portion of the upper middle foot portion 26 from the inside to the outside of the upper 2, and extends from the medial foot side to the lateral foot side along the upper rear foot portion 27 and is fixable to the upper rear foot portion 27. Note that, in the present specification, "a state in which the first pulling-up member 6 extends along the upper rear foot portion 27" means "a state in which the first pulling-up member 6 extends along the extending direction of the tibialis posterior tendon Te of the foot F of the wearer". The first pulling-up member 6 includes a first belt 61, a second belt 62, and a coupling part 63. As a material of the first belt 61 and the second belt 62, for example, a woven fabric, a knitted fabric, synthetic leather, or resin is used.

The first belt 61 is connected to the medial foot side portion of the first support member 5 inside the upper 2, and passes through the medial foot side portion of the upper middle foot portion 26 from the inside to the outside of the upper 2. A part of the first belt 61 is exposed to the outside of the upper 2 through the first through hole 20c. The first belt 61 is fixed to the first support member 5 by stitching, for example.

As illustrated in FIG. 3, a lateral foot side portion of the second belt 62 is fixed to a lateral foot side portion of the upper rear foot portion 27, the second belt 62 extends from the lateral foot side to the medial foot side along the upper rear foot portion 27, and then is folded back at the coupling part 63 and extends to the lateral foot side. The second belt 62 includes a first pulling-up side extending portion 62a and a second pulling-up side extending portion 62b. A lateral foot side portion of the first pulling-up side extending portion 62a is fixed to the lateral foot side portion of the upper rear foot portion 27. The first pulling-up side extending portion 62a extends from the lateral foot side toward the medial foot side to the coupling part 63 along the upper rear foot portion 27. A hook-and-loop fastener 62c is attached to a surface of the first pulling-up side extending portion 62a facing away from the upper rear foot portion 27. A hook-and-loop fastener 62d is attached to a surface of the second pulling-up side extending portion 62b facing the upper rear foot portion 27. The second pulling-up side extending portion 62b can be fixed to and separated from the first pulling-up side extending portion 62a via the hook-and-loop fasteners 62c and 62d. In a state in which the second pulling-up side extending portion 62b is fixed to the first pulling-up side extending portion 62a, the second pulling-up side extending portion 62b extends from the coupling part 63 to the lateral foot side portion of the upper rear foot portion 27.

As illustrated in FIG. 2, the coupling part 63 couples an end portion of the first belt 61 exposed to the outside of the upper 2 and a folded portion of the second belt 62 to each other. A configuration of the coupling part 63 is not particularly limited as long as the first belt 61 and the second belt 62 can be coupled together, but is a ring-shaped member in the present embodiment. In the present embodiment, the end portion of the first belt 61 exposed to the outside of the upper 2 passes through the coupling part 63 and is folded back, and overlapping portions of the first belt 61 are stitched together, whereby the first belt 61 is coupled to the coupling part 63.

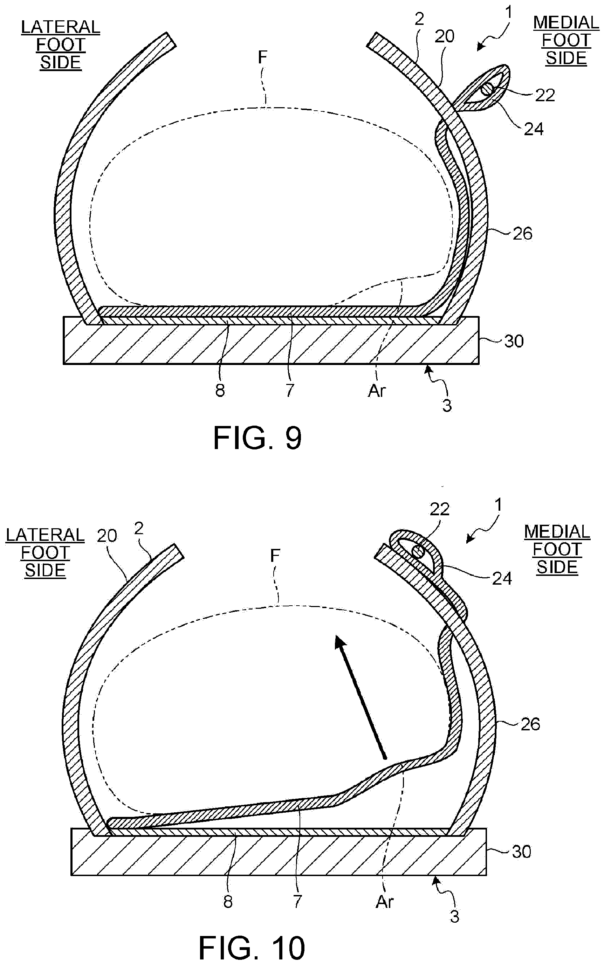

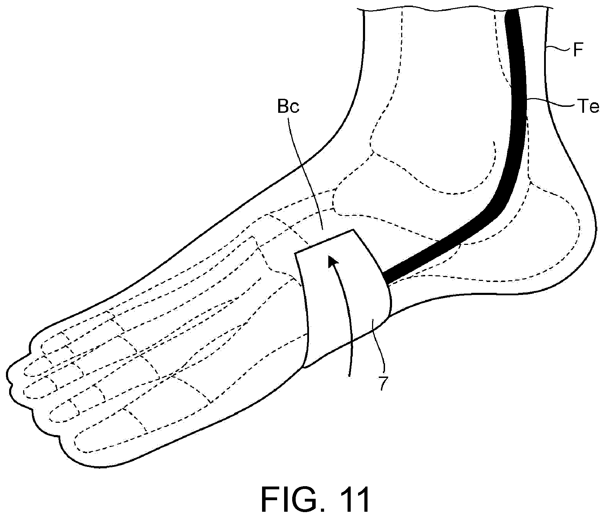

FIG. 9 is a cross-sectional view taken along line IX-IX illustrated in FIG. 4, and is a cross-sectional view illustrating a state before the second support member 7 is pulled up. FIG. 10 is a cross-sectional view illustrating a state after the second support member 7 is pulled up from the state illustrated in FIG. 9. FIG. 11 is a perspective view schematically illustrating a state in which the second support member 7 is overlapped on the foot F. As illustrated in FIGS. 5 and 9, the second support member 7 is disposed in front of the first support member 5 inside the upper 2 and below the medial longitudinal arch Ar, and its lateral foot side portion is fixed to the upper main body 20. As illustrated in FIG. 5, the first support member 5 and the second support member 7 are arranged to be shifted in the front-rear direction not to overlap each other in the vertical direction. As illustrated in FIG. 9, the second support member 7 is disposed below the entire foot F including the medial longitudinal arch Ar. The second support member 7 is disposed in the upper middle foot portion 26. Note that the second support member 7 may be fixed to the footbed 8 or the insole of the sole 3 illustrated in FIG. 9. The second support member 7 is fixed to any of the upper main body 20, the footbed 8, and the insole by stitching, welding, bonding, or a combination thereof. As a material of the second support member 7, for example, a woven fabric, a knitted fabric, synthetic leather, or resin is used. As illustrated in FIG. 5, the width of the second support member 7 in the front-rear direction increases from a connection portion with the shoelace 22 illustrated in FIG. 9 toward the lateral foot side. A plan view shape of the second support member 7 is not particularly limited, but is a trapezoidal shape in the present embodiment. A front end portion, a rear end portion, and a medial foot side end portion of the second support member 7 have a curved shape in the present embodiment, but may have, for example, a linear shape, a waveform shape, or a jagged shape.

As illustrated in FIG. 10, the shoelace 22 is a second pulling-up member connected to the medial foot side portion of the second support member 7 and enabled to apply, to the second support member 7, force for pulling up the second support member 7 in the normal direction of the medial longitudinal arch Ar. In other words, the shoelace 22 is enabled to apply, to the second support member 7, force for pulling up the second support member 7 in a direction in which the second support member 7 is separated from the sole 3. In yet other words, as illustrated in FIG. 11, the shoelace 22 is enabled to apply, to the second support member 7, force for pulling up the second support member 7 upward. Specifically, as illustrated in FIG. 10, when the shoelace 22 is tensioned, the second string passing portion 24 is pulled upward and toward the lateral foot side, and the second support member 7 is pulled up in the normal direction of the medial longitudinal arch Ar to be separated from the sole 3.

Next, effects of the shoe 1 according to the present embodiment will be described.

In the present embodiment, as illustrated in FIGS. 6 and 7, the shoe 1 includes: the first support member 5 disposed below the medial longitudinal arch Ar of the foot F inside the upper 2 and including the lateral foot side portion fixed to the upper 2; and the first pulling-up member 6 connected to the medial foot side portion of the first support member 5 and enabled to apply, to the first support member 5, the force for pulling up the first support member 5 in the direction toward the upper rear foot portion 27 and away from the sole 3. With this configuration, by pulling the first pulling-up member 6, the first support member 5 is pulled up in the direction toward the upper rear foot portion 27 and away from the sole 3, so that falling of the medial longitudinal arch Ar of the foot F can be suppressed. Also by suppressing the falling of the medial longitudinal arch Ar of the foot F, excessive extension of the tibialis posterior tendon Te can be suppressed, and a burden on the tibialis posterior tendon Te can be reduced. In addition, since the direction in which the first support member 5 is pulled up is along the extending direction of the tibialis posterior tendon Te, it is possible to suppress the excessive extension of the tibialis posterior tendon Te and reduce the burden on the tibialis posterior tendon Te.

In the present embodiment, as illustrated in FIGS. 9 and 10, the shoe 1 includes: the second support member 7 disposed in front of the first support member 5 inside the upper 2 and disposed below the medial longitudinal arch Ar, and including the lateral foot side portion fixed to the upper 2; and the shoelace 22 connected to the medial foot side portion of the second support member 7 and enabled to apply, to the second support member 7, the force for pulling up the second support member 7 in the normal direction of the medial longitudinal arch Ar. With this configuration, since the second support member 7 is pulled up in the normal direction of the medial longitudinal arch Ar by tensioning the shoelace 22, the falling of the medial longitudinal arch Ar of the foot F can be further suppressed.

In the present embodiment, as illustrated in FIGS. 2 and 3, the first pulling-up member 6 includes: the first belt 61 connected to the medial foot side portion of the first support member 5 and passing through the medial foot side portion of the upper middle foot portion 26 from the inside to the outside of the upper 2; the second belt 62 including the lateral foot side portion fixed to the lateral foot side portion of the upper rear foot portion 27, extending from the lateral foot side to the medial foot side along the upper rear foot portion 27, and then folded back and extending to the lateral foot side; and the coupling part 63 that couples the first belt 61 and the folded portion of the second belt 62 to each other. As described above, since the first pulling-up member 6 has a structure independent of the shoelace 22 and the adjuster belt 4, a degree of pulling-up of the first support member 5 can be independently adjusted without considering a degree of tightening of the upper main body 20.

In the present embodiment, as illustrated in FIG. 3, one second string passing portion 24 of the plurality of string passing portions is provided at the medial foot side portion of the second support member 7, and the shoelace 22 alternately passes through the first string passing portions 23 and the second string passing portion 24 provided at one of the side edges of the opening 20b and the first string passing portions 23 provided at another of the side edges of the opening 20b. With this configuration, by tensioning the shoelace 22, tightening of the upper main body 20 and pulling-up of the second support member 7 can be performed at the same time, so that a burden on the wearer can be reduced.

In the present embodiment, as illustrated in FIG. 5, the width of the first support member 5 in the front-rear direction increases from the connection portion with the first pulling-up member 6 toward the lateral foot side, so that it is possible to alleviate contact of the first support member 5 with the foot F of the wearer. That is, since a contact area between the foot F of the wearer and the shoe 1 increases toward the lateral foot side, it is possible to alleviate the contact of the first support member 5 with the foot F of the wearer by increasing a contact area between the foot F and the first support member 5 toward the lateral foot side. In addition, in the present embodiment, as illustrated in FIG. 5, the width of the second support member 7 in the front-rear direction increases from the connection portion with the shoelace 22 illustrated in FIG. 2 toward the lateral foot side, so that it is possible to alleviate contact of the second support member 7 with the foot F of the wearer. That is, since the contact area between the foot F of the wearer and the shoe 1 increases toward the lateral foot side, it is possible to alleviate the contact of the second support member 7 with the foot F of the wearer by increasing a contact area between the foot F and the second support member 7 toward the lateral foot side.

In the present embodiment, as illustrated in FIGS. 7 and 10, the sole 3 includes the outsole 30 and the footbed 8 placed on the outsole 30 inside the upper 2, and the first support member 5 and the second support member 7 are arranged on the footbed 8. With this configuration, the first support member 5 and the second support member 7 can be directly brought into contact with the foot F, and the first support member 5 and the second support member 7 can be deformed along a shape of the foot F of each wearer. The first support member 5 and the second support member 7 can therefore be favorably brought into contact with the foot F of the wearer.

### (Second embodiment)

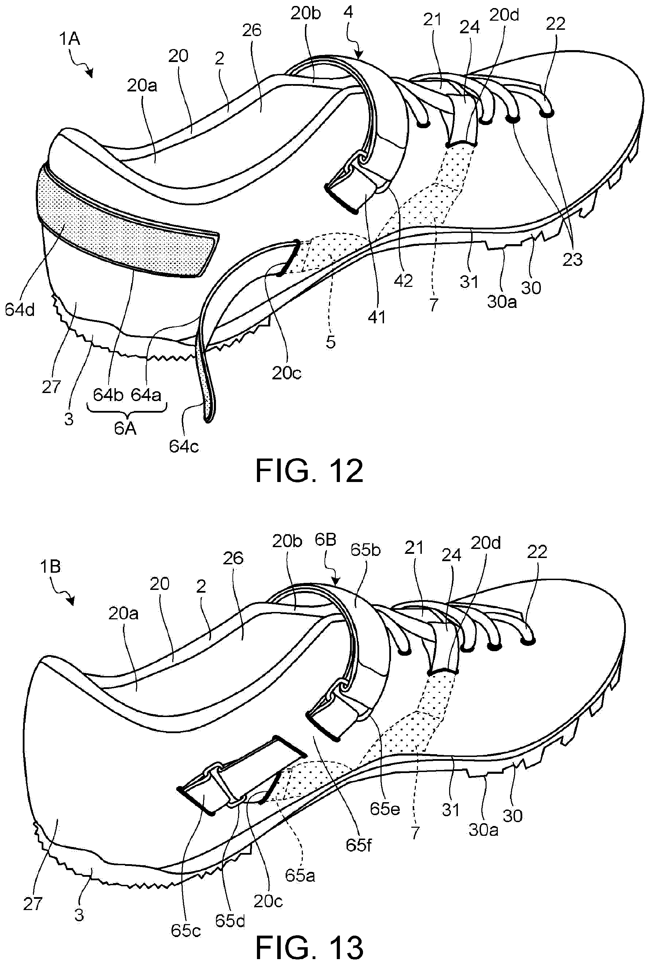

FIG. 12 is a perspective view of a shoe 1A according to a second embodiment of the present invention. The shoe 1A according to the second embodiment is different from the shoe 1 according to the first embodiment in a configuration of a first pulling-up member 6A. Note that, in the second embodiment, the same reference numerals are given to portions overlapping with the first embodiment described above, and description thereof is omitted.

The first pulling-up member 6A passes through the medial foot side portion of the upper middle foot portion 26 from the inside to the outside of the upper 2, and extends along the upper rear foot portion 27 and is fixable to the upper rear foot portion 27. The first pulling-up member 6A includes one belt 64a and a belt fixing portion 64b. As a material of the belt 64a and the belt fixing portion 64b, for example, a woven fabric, a knitted fabric, synthetic leather, or resin is used.

The belt 64a is connected to the medial foot side portion of the first support member 5 inside the upper 2, and passes through the medial foot side portion of the upper middle foot portion 26 from the inside to the outside of the upper 2. A part of the belt 64a is exposed to the outside of the upper 2 through the first through hole 20c. The belt 64a is fixed to the first support member 5 by stitching, for example. A hook-and-loop fastener 64c is attached to a surface of the belt 64a facing the upper rear foot portion 27.

The belt fixing portion 64b is fixed to the upper rear foot portion 27 and extends in the left-right direction along the upper rear foot portion 27. A hook-and-loop fastener 64d is attached to a surface of the belt fixing portion 64b facing away from the upper rear foot portion 27. The belt 64a can be fixed to and separated from the belt fixing portion 64b via the hook-and-loop fasteners 64c and 64d. In a state in which the belt 64a is fixed to the belt fixing portion 64b, the belt 64a extends from the medial foot side to the lateral foot side along the upper rear foot portion 27. The present embodiment can have effects similar to those of the first embodiment described above. Note that the first support member 5 and the belt 64a may be integrally formed.

### (Third embodiment)

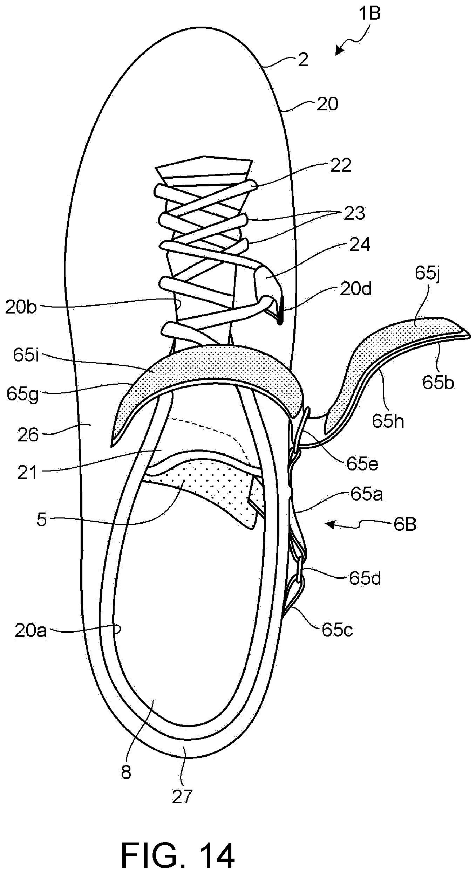

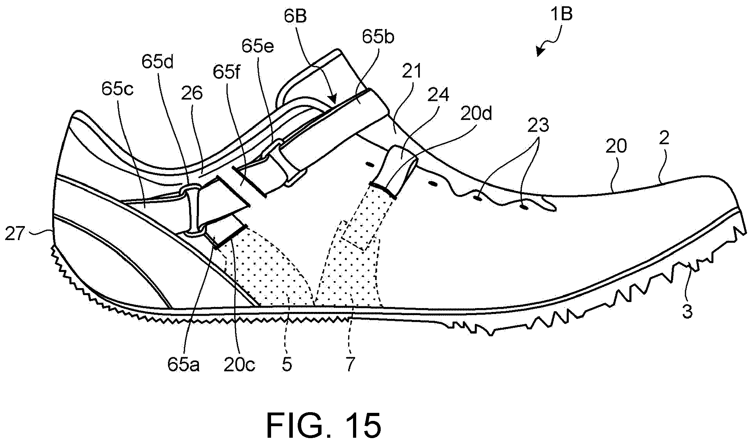

FIG. 13 is a perspective view of a shoe 1B according to a third embodiment of the present invention. FIG. 14 is a plan view of the shoe 1B according to the third embodiment. FIG. 15 is a side view of the medial foot side of the shoe 1B according to the third embodiment. The shoe 1B according to the third embodiment is different from the shoe 1 according to the first embodiment in a configuration of a first pulling-up member 6B. Note that, in the third embodiment, the same reference numerals are given to portions overlapping with the first embodiment described above, and description thereof is omitted.

As illustrated in FIG. 13, the first pulling-up member 6B includes a first belt 65a, a second belt 65b, a fixed belt 65c, a connecting part 65d, and a coupling part 65e. The fixed belt 65c and the connecting part 65d are located obliquely above and in the rear from the first through hole 20c, in the medial foot side portion of the upper middle foot portion 26. The coupling part 65e is located obliquely upward and in front from the first through hole 20c and in the rear from the second through hole 20d, in the medial foot side portion of the upper middle foot portion 26.

The first belt 65a is connected to the medial foot side portion of the first support member 5 inside the upper 2, and passes through the medial foot side portion of the upper middle foot portion 26 from the inside to the outside of the upper 2. A part of the first belt 65a is exposed to the outside of the upper 2 through the first through hole 20c. The first belt 65a extends from the first through hole 20c toward the connecting part 65d, is then folded back at the connecting part 65d, and extends toward the coupling part 65e. A portion of the first belt 65a from the first through hole 20c to the connecting part 65d extends along the extending direction of the tibialis posterior tendon Te illustrated in FIG. 8. A part of a portion of the first belt 65a extending between the connecting part 65d and the coupling part 65e passes between an outer layer and an inner layer of the upper main body 20. The outer layer of the upper main body 20 serves as a deflection suppressing portion 65f that suppresses deflection of the first belt 65a toward the medial foot side.

A first end of the fixed belt 65c is fixed to the upper middle foot portion 26. A second end of the fixed belt 65c is connected to the connecting part 65d.

The connecting part 65d connects the second end of the fixed belt 65c and a folded portion of the first belt 65a to each other. A configuration of the connecting part 65d is not particularly limited as long as the fixed belt 65c and the first belt 65a can be connected to each other, but is a ring-shaped member in the present embodiment. In the present embodiment, the second end of the fixed belt 65c is passed through the connecting part 65d and folded back, and overlapping portions of the fixed belt 65c are stitched together, whereby the fixed belt 65c is connected to the connecting part 65d.

The second belt 65b is a member corresponding to the adjuster belt 4 of the first embodiment described above. The second belt 65b is enabled to apply, to the upper main body 20, tightening force for pulling the medial foot side portion and the lateral foot side portion of the upper middle foot portion 26 toward each other. As illustrated in FIG. 14, the lateral foot side portion of the second belt 65b is fixed to the lateral foot side portion of the upper middle foot portion 26, The second belt 65b extends from the lateral foot side portion to the medial foot side portion of the upper middle foot portion 26, and then is folded back at the coupling part 65e and extends to the lateral foot side. The second belt 65b includes a first extending portion 65g and a second extending portion 65h. A lateral foot side portion of the first extending portion 65g is fixed to the lateral foot side portion of the upper middle foot portion 26. The first extending portion 65g extends from the lateral foot side portion toward the medial foot side portion of the upper middle foot portion 26 to the coupling part 65e. A hook-and-loop fastener 65i is attached to a surface of the first extending portion 65g facing away from the upper middle foot portion 26. A hook-and-loop fastener 65j is attached to a surface of the second extending portion 65h facing the upper middle foot portion 26. The second extending portion 65h can be fixed to and separated from the first extending portion 65g via the hook-and-loop fasteners 65i and 65j. In a state in which the second extending portion 65h is fixed to the first extending portion 65g, the second extending portion 65h extends from the coupling part 65e to the lateral foot side portion of the upper middle foot portion 26.

As illustrated in FIG. 13, the coupling part 65e couples an end portion of the first belt 65a exposed to the outside of the upper 2 and a folded portion of the second belt 65b to each other. A configuration of the coupling part 65e is not particularly limited as long as the first belt 65a and the second belt 65b can be coupled together, but is a ring-shaped member in the present embodiment. In the present embodiment, the end portion of the first belt 65a exposed to the outside of the upper 2 passes through the coupling part 65e and is folded back, and overlapping portions of the first belt 65a are stitched together, whereby the first belt 65a is coupled to the coupling part 65e.

The present embodiment can have effects similar to those of the first embodiment described above. In addition, in the present embodiment, as illustrated in FIGS. 13 to 15, the first pulling-up member 6B includes the first belt 65a connected to the medial foot side portion of the first support member 5 and passing through the medial foot side portion of the upper middle foot portion 26 from the inside to the outside of the upper 2. In addition, the first pulling-up member 6B includes the second belt 65b including the lateral foot side portion fixed to the lateral foot side portion of the upper middle foot portion 26, extending from the lateral foot side portion to the medial foot side portion of the upper middle foot portion 26, and then folded back and extending to the lateral foot side. The second belt 65b is enabled to apply, to the upper 2, tightening force for pulling the medial foot side portion and the lateral foot side portion of the upper middle foot portion 26 toward each other. Furthermore, the first pulling-up member 6B includes the coupling part 65e that couples the first belt 65a and the folded portion of the second belt 65b to each other. With these configurations, a structure is made in which the first support member 5 is pulled up in conjunction with operation of tightening the second belt 65b. Thus, by tightening the second belt 65b, tightening of the upper main body 20 and pulling-up of the first support member 5 can be performed at the same time, so that the burden on the wearer can be reduced.

### (Fourth embodiment)

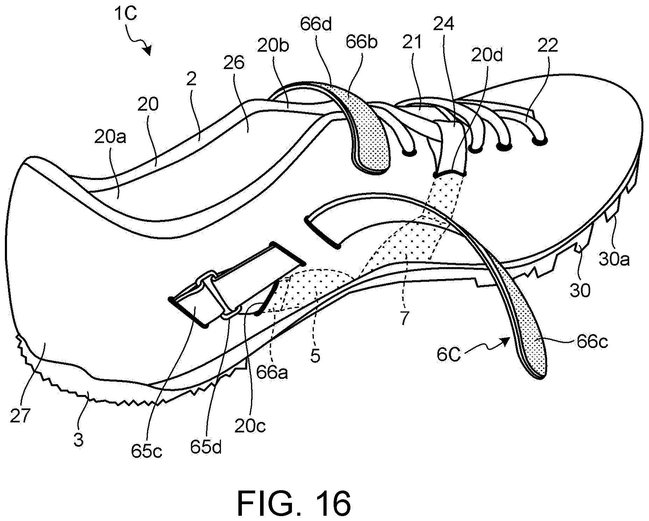

FIG. 16 is a perspective view of a shoe 1C according to a fourth embodiment of the present invention. The shoe 1C according to the fourth embodiment is different from the shoe 1B according to the third embodiment in a configuration of a first pulling-up member 6C. Note that, in the fourth embodiment, the same reference numerals are given to portions overlapping with the third embodiment described above, and description thereof is omitted.

The first pulling-up member 6C passes through the medial foot side portion of the upper middle foot portion 26 from the inside to the outside of the upper 2, extends from the medial foot side portion to the lateral foot side portion of the upper middle foot portion 26, and is fixable to the upper middle foot portion 26. The first pulling-up member 6C includes one belt 66a, the fixed belt 65c, the connecting part 65d, and a belt fixing portion 66b. Configurations of the fixed belt 65c and the connecting part 65d are similar to those of the third embodiment described above.

The belt 66a is connected to the medial foot side portion of the first support member 5 inside the upper 2, and passes through the medial foot side portion of the upper middle foot portion 26 from the inside to the outside of the upper 2. A part of the belt 66a is exposed to the outside of the upper 2 through the first through hole 20c. The belt 66a extends from the first through hole 20c toward the connecting part 65d, is then folded back at the connecting part 65d, and extends toward the belt fixing portion 66b. A portion of the belt 66a from the first through hole 20c to the connecting part 65d extends along the extending direction of the tibialis posterior tendon Te illustrated in FIG. 8. A hook-and-loop fastener 66c is attached to a surface of the belt 66a facing the upper middle foot portion 26.

The belt fixing portion 66b is fixed to the upper middle foot portion 26, and extends in the left-right direction across the opening 20b in the upper middle foot portion 26. A hook-and-loop fastener 66d is attached to a surface of the belt fixing portion 66b facing away from the upper middle foot portion 26. The belt 66a can be fixed to and separated from the belt fixing portion 66b via the hook-and-loop fasteners 66c and 66d. In a state in which the belt 66a is fixed to the belt fixing portion 66b, the belt 66a extends from the medial foot side portion to the lateral foot side portion of the upper middle foot portion 26. The present embodiment can have effects similar to those of the first and third embodiments described above. Note that, the first support member 5 and the belt 66a may be integrally formed.

The configuration described in each of the above embodiments illustrates an example of the content of the present invention, and can be combined with another known technique, and a part of the configuration can be omitted or changed without departing from the gist of the present invention. In the first to fourth embodiments, the second support member 7 is included, but may be omitted. In a case where the second support member 7 is omitted, the size of the first support member 5 may be larger than the illustrated example. In the first to fourth embodiments described above, the first support member 5 and the second support member 7 are arranged on the footbed 8, but may be arranged under the footbed 8.

The shoe according to the present invention has an effect of reducing the burden on the tibialis posterior tendon while suppressing the falling of the medial longitudinal arch.

## Claims

1. A shoe (1, 1A, 1B, 1C) comprising:
an upper (2) in which an upper middle foot portion (26) covering a middle foot portion of a foot of a wearer, an upper rear foot portion (27) covering a heel portion of the foot, and a foot insertion opening (20a) for inserting the foot are formed;
a sole (3) located below the upper (2);
a first support member (5) disposed below a medial longitudinal arch (Ar) of the foot inside the upper (2) and including a lateral foot side portion fixed to the upper (2) or the sole (3); and
a first pulling-up member (6,6A,6B,6C) connected to a medial foot side portion of the first support member (5) and enabled to apply, to the first support member (5), force for pulling up the first support member (5) in a direction toward the upper rear foot portion (27) and away from the sole (3).

2. The shoe (1,1A) according to claim 1, wherein the first pulling-up member (6,6A) passes through a medial foot side portion of the upper middle foot portion (26) from an inside to an outside of the upper (2), and extends from a medial foot side to a lateral foot side along the upper rear foot portion (27) and is fixable to the upper rear foot portion (27).

3. The shoe (1) according to claim 1 or 2, wherein
the first pulling-up member (6) includes:
a first belt (61) connected to the medial foot side portion of the first support member (5) and passing through a medial foot side portion of the upper middle foot portion (26) from an inside to an outside of the upper (2);
a second belt (62) including a lateral foot side portion fixed to a lateral foot side portion of the upper rear foot portion (27), extending from a lateral foot side to a medial foot side along the upper rear foot portion (27), and then folded back and extending to the lateral foot side; and
a coupling part (63) that couples the first belt (61) and a folded portion of the second belt (62) to each other.

4. The shoe (1B,1C) according to claim 1, wherein the first pulling-up member (6B,6C) passes through a medial foot side portion of the upper middle foot portion (26) from an inside to an outside of the upper (2), and extends from the medial foot side portion to a lateral foot side portion of the upper middle foot portion (26) and is fixable to the upper middle foot portion (26).

5. The shoe (1B) according to claim 1 or 4, wherein
the first pulling-up member (6B) includes:
a first belt (65a) connected to the medial foot side portion of the first support member (5) and passing through a medial foot side portion of the upper middle foot portion (26) from an inside to an outside of the upper (2);
a second belt (65b) including a lateral foot side portion fixed to a lateral foot side portion of the upper middle foot portion (26), extending from the lateral foot side portion to the medial foot side portion of the upper middle foot portion (26), then folded back and extending to a lateral foot side, and enabled to apply, to the upper (2), tightening force for pulling the medial foot side portion and the lateral foot side portion of the upper middle foot portion (26) toward each other; and
a coupling part (65e) that couples the first belt (65a) and a folded portion of the second belt (65b) to each other.

6. The shoe (1,1A,1B,1C) according to any one of claims 1 to 5, wherein
the upper (2) includes: a second support member (7) disposed in front of the first support member (5) inside the upper (2) and disposed below the medial longitudinal arch (Ar), and including a lateral foot side portion fixed to the upper (2) or the sole (3); and
a second pulling-up member (22) connected to a medial foot side portion of the second support member (7) and enabled to apply, to the second support member (7), force for pulling up the second support member (7) in a normal direction of the medial longitudinal arch (Ar).

7. The shoe (1,1A,1B,1C) according to claim 6, wherein
an opening (20b) communicating with the foot insertion opening (20a) and extending in front from the foot insertion opening (20a) is formed in the upper (2),
a plurality of string passing portions (23,24) separated from each other in a front-rear direction is provided at left and right side edges of the opening (20b),
one of the plurality of string passing portions (23,24) is provided in the medial foot side portion of the second support member (7), and
the second pulling-up member (22) is a shoelace that alternately passes through the string passing portions (23,24) provided at one of the side edges of the opening (20b) and the string passing portions (23,24) provided at another of the side edges of the opening (20b).

8. The shoe (1,1A,1B,1C) according to any one of claims 1 to 7, wherein a width of the first support member (5) in a front-rear direction increases from a connection portion with the first pulling-up member (6,6A,6B,6C) toward a lateral foot side.

9. The shoe (1,1A,1B,1C) according to any one of claims 1 to 8, wherein
the sole (3) includes an outsole (30) and a footbed (8) placed on the outsole (30), and
the first support member (5) is disposed on the footbed (8).
